# EUROPÄISCHE PATENTANMELDUNG

(11) **EP 1 647 527 A1**
(43) Veröffentlichungstag der Anmeldung: **19.04.2006**
(21) Anmeldenummer: 05020289.4
(22) Anmeldetag: 16.09.2005
(51) Int. Cl.: C02F 1/50

(54) **Verfahren zur Vermeidung oder wenigstens teilweisen Vermeidung von Mikroorganismen auf Wasserbenetzten Oberflächen in Kühltürmen aller Bauarten**

(30) Priorität: 18.10.2004 DE 102004050613
(71) Anmelder: Jäggi/Güntner (Schweiz) AG, 4632 Trimbach (CH)
(72) Erfinder: Dierks, Gert, 5621 Zufikon (CH)
(74) Vertreter: Volpert, Marcus

(57) **Zusammenfassung**

Die Erfindung betrifft ein Verfahren zur Vermeidung oder wenigstens teilweisen Vermeidung der Bildung von Mikroorganismen auf wasserbenetzten Oberflächen in Kühltürmen aller Bauarten. Bei derartigen Kühltürmen soll die Bildung von Mikroorganismen und insbesondere von Legionellen auf den wasserbenetzten Oberflächen vermieden werden. Dies geschieht dadurch, dass die wasserbenetzten Oberflächen mit antimikrobielle Wirkungen entfaltenden Stoffen unter Anwendung der Lackier- und Nanotechnologie beschichtet werden

## Beschreibung

Die Erfindung betrifft ein Verfahren zur Vermeidung oder wenigstens teilweisen Vermeidung der Bildung von Mikroorganismen auf wasserbenetzten Oberflächen und Einbauten in Kühltürmen aller Bauarten sowie zum Abbau solcher Mikroorganismen.

Antimikrobielle Stoffe, insbesondere Kunststoffe, werden seit längerer Zeit in der Medizin-, Labor- und Klimatechnik alswachstumshemmer gegen Bakterienbefall sowie Hefen, Schimmelpilze o.dgl. verwendet. Derartige antimikrobielle Wirkstoffe sind auch bereits als Bestandteil von Trägermaterialien bekannt, aus denen sie, beispielsweise in Form von Silberionen, zur Bekämpfung von für den Menschen schädlichen Mikroorganismen bzw. zum Abbau derselben abgegeben werden.

In diesem Zusammenhang ist auch bereits die antimikrobielle Wirkung der Oberflächen von Bauteilen bekannt geworden, auf denen Beschichtungen auf Silberbasis aufgebracht werden, oder bei denen Kunststoffe zum Einsatz kommen, welche mit einem Compound versetzt sind, das bei Benetzung mit Wasser permanent Silberionen freisetzt, die eine starke antimikrobielle Wirkung entfalten und das Wachstum sowie die Migration von Bakterien, Hefe, Schimmel und Pilzen verhindem, so dass der Zusatz von Chemikalien wie z.B. Bioziden zum Kühlwasser oder zum Benetzungs- und Besprühungswasser nicht mehr erforderlich ist.

Basierend auf der Nanotechnologie existieren oberflächliche Wirkstoffkombinationen, die anti-bakterielle Wirkstoffe, welche einen hohen Wirkungsgrad haben, freisetzen, wobei das auf die Anwendung der Nanotechnologie beruhende Beschichtungssystem die Vorteile anorganischer, biozider Wirkstoffe nutzt und mit den überragenden Eigenschaften einer extrem großen spezifischen Oberfläche und Reaktivität von Nanopartikeln so kombiniert, dass eine kontinuierliche anti-mikrobielle Langzeitwirkung erreicht wird.

Es hat sich nun gezeigt, dass Rieseleinsätze in Kühltürmen, wie in der DE-PS 32 34 251 und der DE-OS 195 19 924 beschrieben, die beispielsweise in zwangsbelüfteten bzw. auch in naturbelüfteten geschlossenen und offenen Kühltürmen der in der DE-PS 196 51 848, der DE-OS 29 30 567, der DE-OS 101 58 049 sowie der DE-OS 197 39 724 beschriebenen Art Verwendung finden, einem erheblichen mikrobiologischen Wachstum ausgesetzt sind, das wegen des Legionellenproblems erhebliche für den Menschen schädliche gesundheitliche Auswirkungen haben kann. Hauptverursacher sind namentlich Legionellen in offenen Kühlkreisläufen von Kühltürmen, die dadurch gefährlich sind, dass beim Versprühen des Kühlwassers kleinste Tröpfchen, sogenannte Aerosole, zusammen mit der Kühlluft ausgebracht werden. Wenn diese mit Legionellen kontaminiert sind, können sie in die menschliche Lunge gelangen und dann die sogenannte Legionärskrankheit auslösen.

Um das mikrobiologische Wachstum und damit die Legionellenbildung zu verhindern, wird heutzutage das Kühlwasser mit chemischen Substanzen konditioniert. Diese Verfahrensweise ist jedoch wegen des entstehenden Abwassers, die sogenannte Abschlämmung, das bei jedem Kühlturm anfällt, infolge Umweltbelastung nicht unproblematisch.

Die Aufgabe der Erfindung besteht deshalb darin, mit Hilfe der vorhandenen bekannten Erkenntnissen und konstruktiven Maßnahmen die Bildung von Mikroorganismen und insbesondere von Legionellen auf wasserbenetzten Wärmeaustauscheroberflächen usw.mit einem wirtschaftlich vertretbaren Aufwand ganz oder wenigstens teilweise zu vermeiden und damit die gesetzlich vorgegebenen Anforderungen zu erfüllen und darüber hinaus die bewährten geschlossenen und offenen Kühlturmkonstruktionen, zu denen auch Hydridkühler gerechnet werden, weiter benutzbar machen.

Diese Aufgabe wird erfindungsgemäß dadurch gelöst, dass die wasserbenetzten Oberflächen der Kühltürme wie oben erwähnt mit anti-mikrobielle Wirkungen entfaltenden Stoffen unter Anwendung der Lackier- u. Nanotechnologie beschichtet werden.

Der erfinderische Grundgedanke ist also, dass man die antimikrobiellen Eigenschaften von Schichten oder Werkstoffen für die massgebenden Oberflächen von Kühltürmen aller Bauarten anwendet.

Als Beschichtungsmaterialien lassen sich insbesondere Biozidpartikel und in diesem Zusammenhang Silberionen oder andere biozide Additive verwenden, die auf einer Trägersubstanz oberflächlich implementiert sind und eine Langzeitwirkung entfalten, indem sie aus dieser Trägersubstanz nicht ausgewaschen werden.

In der Zeichnung sind zwei Ausführungsbeispiele in Form von schematisch dargestellten, zwangsbelüfteten Kühltürmen gezeigt, die mit Einbauten arbeiten, deren Oberflächen mit anti-mikrobiell wirkenden Stoffen in Form von bioziden Partikeln nanobeschichtet sind, d.h. eine Schichtdicke von beispielsweise kleiner als 10 nm aufweisen.

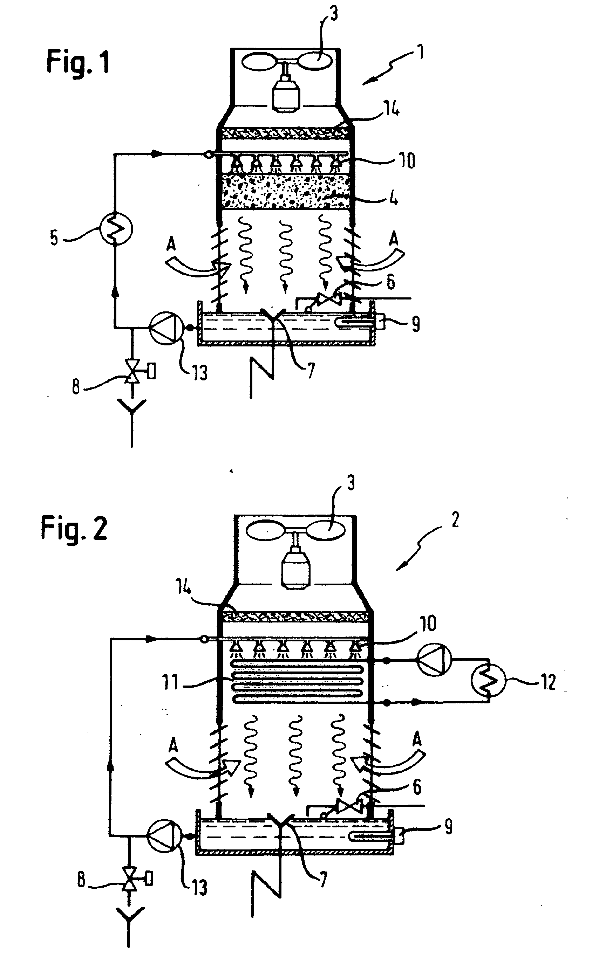

Fig. 1 zeigt diesbezüglich einen offenen Kühlturm 1, dessen Zwangsbelüftung mit Hilfe eines Ventilators 3 erfolgt, unter dem ein Tropfenabscheider 14 sowie eine Füllkörperpackung 4 angeordnet sind, deren Oberflächen beide die anti-mikrobielle Beschichtung aufweisen. Ebenso die Kühlturmwände.

Die Frischwasserzufuhr erfolgt über ein Zulaufventil 6 und eine Wärmequelle 5 mit sich daran anschließendem Düsenstock 10, über den das Kühlwasser über zahlreiche Düsen auf die Füllkörper 4 der Rieselpackung verteilt wird, die eine große Oberfläche aufweisen, welche die seitlich gemäß den dargestellten Pfeilen A in den Kühlturm eintretende Luft, die von dem Ventilator 3 durch die Rieselpackung mit den Füllkörpern 4 hindurchgesaugt wird, abkühlt.

Der Kühlturm von Fig. 1 ist wie üblich mit einem Überlauf 7, einem Abschlämmventil 8, einer Frostschutzheizung 9 und einer internen Umlaufspülwasserpumpe 13 versehen.

Der in Fig. 2 schematisch dargestellte, zwangsbelüftete Kühlturm ist im Gegensatz zu dem Kühlturm von Fig. 1 geschlossen, was bedeutet, dass das über den Düsenstock 10 abgegebene Benetzungsumlaufwasser auf ein beripptes oder unberipptes Rohrbündelregister 11 fällt, dessen Oberfläche wie die Füllkörper 4 der Rieselpackung des offenen Kühlturms von Fig. 1 mit einer antimikrobiellen Beschichtung ausgeführt ist. Im Übrigen entspricht der Kühlturmaufbau in seinen wesentlichen Elementen demjenigen von Fig. 1, was bedeutet, dass ebenfalls ein Tropfenabscheider 14 vorhanden ist, der eine antimikrobielle Beschichtung aufweist, so dass die mit Hilfe des Ventilators 3 in den Kühlturm entsprechend den Pfeilen A eingesaugte Kühlluft, mit der kleinste Tröpfchen, die sogenannten Aerosole, ausgetragen werden, nicht kontaminiert ist.

Es versteht sich, dass die in der Zeichnung dargestellten Kühltürme nur als Beispiel für Kühltürme an sich beliebiger Konstruktion dienen sollen, bei denen die Bildung von Mikroorganismen, insbesondere der sehr gefährlichen Legionellen, verhindert werden soll.

## Patentansprüche

1. Verfahren zur Vermeidung oder wenigstens teilweisen Vermeidung der Bildung von Mikroorganismen auf wasserbenetzten Oberflächen in Kühltürmen aller Bauarten, **dadurch gekennzeichnet, dass** die mit Wasser benetzten Oberflächen mit antimikrobielle Wirkungen entfaltenden Stoffen unter Anwendung der Lackier- u. Nanotechnologie beschichtet werden.

2. Verfahren nach Anspruch 1, **dadurch gekennzeichnet, dass** die antimikrobielle Wirkung der Beschichtung durch antimikrobielle Additive in der Beschichtung erzeugt wird.

3. Verfahren nach Anspruch 1 oder 2, **dadurch gekennzeichnet, dass** die antimikrobielle Wirkung der Beschichtung mittels Silberionen erzeugt wird.

4. Verfahren nach einem der Ansprüche 1 bis 3, **dadurch gekennzeichnet, dass** die durchschnittliche Dicke der Beschichtung im Nanobereich liegt.

5. Verwendung von antimikrobielle Wirkungen entfaltenden Stoffen, insbesondere Kunststoffen, und Lackierungen mit entsprechenden antimikrobiellen Coumpounds, und Materialien zur Vermeidung, Wachstumshemmung sowie zum Abbau von Mikroorganismen auf wasserbenetzten Oberflächen in Kühltürmen und Wärmeübertragem aller Bauarten.
